(19) Europäisches Patentamt
European Patent Office
Office européen des brevets

(11) **EP 4 570 785 A1**

(12) **EUROPEAN PATENT APPLICATION**
published in accordance with Art. 153(4) EPC

(43) Date of publication:
**18.06.2025  Bulletin 2025/25**

(21) Application number: 23853015.8

(22) Date of filing: **10.08.2023**

(51) International Patent Classification (IPC):
**C07C 263/10** (2006.01)    **C07C 263/18** (2006.01)
**G02B 1/04** (2006.01)

(52) Cooperative Patent Classification (CPC):
**C07C 263/10; C07C 263/18; G02B 1/04**

(86) International application number:
**PCT/KR2023/011815**

(87) International publication number:
**WO 2024/035146 (15.02.2024 Gazette 2024/07)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  10.08.2022  KR 20220100078
                09.08.2023  KR 20230104263

(71) Applicant: **Hanwha Solutions Corporation
Jung-gu
Seoul 04541 (KR)**

(72) Inventors:
• **SIM, Yujin
  Daejeon 34128 (KR)**
• **PARK, Jongseong
  Daejeon 34128 (KR)**
• **PARK, Juyoung
  Daejeon 34128 (KR)**
• **WOO, Eun Ji
  Daejeon 34128 (KR)**

(74) Representative: **Berggren Oy
P.O. Box 16
Eteläinen Rautatiekatu 10A
00101 Helsinki (FI)**

(54) **PREPARATION METHOD OF ISOCYANATE COMPOSITION AND ISOCYANATE COMPOSITION**

(57)  Provided are a method of preparing a high-purity isocyanate composition, and an isocyanate composition.

**EP 4 570 785 A1**

**Description**

[Technical Field]

Cross-reference to Related Application

The present application is based on, and claims priority from, Korean Patent Application Nos. 10-2022-0100078 and 10-2023-0104263, filed on August 10, 2022 and August 9, 2023, respectively, the disclosures of which are hereby incorporated by reference herein in their entirety.

The present invention relates to a method of preparing a high-purity isocyanate composition, and an isocyanate composition.

[Background Art]

Isocyanate compounds are highly valuable compounds that are utilized not only in the chemical and resin industries, but also as raw materials for fine chemical products including optical materials. A representative example of the isocyanate compounds is xylylene diisocyanate (hereinafter, referred to as XDI), of which demand is increasing as a high value-added chemical material as a raw material for advanced optical lenses.

Generally, isocyanate is prepared by a phosgenation method of reacting a raw material amine with phosgene. In this regard, the phosgenation reaction that forms aromatic isocyanate is an endothermic reaction, and thus continuous heating and maintenance of a high temperature are required during the reaction to increase the yield of the desired product.

However, since isocyanates such as XDI generally have high reactivity with the amino group, side reactions occur in many cases, and impurities generated through the side reactions make it difficult to use the isocyanates as fine chemical products in fields of optical materials, which require high purity.

Therefore, after the reaction, a purification process is essential to obtain the desired product with high purity. However, as the exposure time at high temperatures in the purification step increases, additional by-products are generated, which causes a problem of reducing the yield of the desired product. Accordingly, it is necessary to study a purification method of improving the yield of the desired product.

[Disclosure]

[Technical Problem]

There are provided a method of preparing a high-purity isocyanate composition by adding a stabilizer in a purification step when producing an isocyanate using phosgene, and an isocyanate composition.

[Technical Solution]

There is provided a method of preparing an isocyanate composition, the method including a reaction step of preparing a composition including an isocyanate by reacting an amine-based compound or a salt thereof with phosgene; and a purification step of separating the isocyanate from the composition, wherein the purification step is performed in the presence of a stabilizer.

Further, there is provided an isocyanate composition including an isocyanate, an isocyanate oligomer, and a stabilizer.

Further, there is provided a polyisocyanate composition including a polyisocyanate which is formed by a polymerization reaction of the isocyanate composition of the present invention and a polyvalent thiol.

Further, there is provided an optical article including the polyisocyanate composition of the present invention.

**In** the present invention, the terms "the first", "the second", and the like are used to describe a variety of components, and these terms are merely employed to differentiate a certain component from other components.

Further, the terms used in this description are just for explaining exemplary embodiments and it is not intended to restrict the present invention.

The singular expression may include the plural expression unless it is differently expressed contextually.

It must be understood that the term "comprise", "equipped", or "have" in the present description is only used for designating the existence of characteristics taken effect, numbers, steps, components, or combinations thereof, and do not exclude one or more different characteristics, numbers, steps, components, combinations thereof, or possibility of addition thereof.

While the present invention is susceptible to various modifications and alternative forms, specific embodiments will be illustrated and described in detail as follows. It should be understood, however, that the description is not intended to limit the present invention to the particular forms disclosed, but on the contrary, the intention is to cover all modifications, equivalents, and alternatives falling within the spirit and scope of the invention.

In this specification, a composition including isocyanate means a mixture including an isocyanate prepared by reacting an amine-based compound or a salt thereof with phosgene, unreacted raw materials, by-products, and other stabilizers, etc.

The present invention provides a method of preparing an isocyanate composition, the method including a reaction step of preparing a composition including an isocyanate by reacting an amine-based compound or a salt thereof with phosgene; and a purification step of separating the isocyanate from the composition, wherein the purification step is performed in the presence of a stabilizer.

Commonly, preparation of isocyanate using the phosgenation reaction is carried out by a reaction of amine and phosgene, and at this time, monoisocyanate and the like are generated as by-products.

For example, in the case of xylylene diisocyanate (XDI) which is an isocyanate, when xylylene diamine and phosgene undergo a phosgenation reaction, phosgene reacts with an amino group of the amine to release hydrogen chloride and to form carbamoyl chloride.

The carbamoyl group of the formed carbamoyl chloride reacts to form an isocyanate group while further releasing hydrogen chloride, thereby forming xylylene diisocyanate.

After completion of the reaction, the produced hydrogen chloride and remaining phosgene are generally removed together with inert gas by distillation, and as a result, they are separated from the produced xylylene diisocyanate.

In this regard, since the amino group in the amine, i.e., xylylene diamine is highly reactive, it causes side reactions during synthesis. In particular, through the side reactions, by-products including monoisocyanate, etc. such as ethylbenzyl isocyanate (EBI) and chloromethylbenzyl isocyanate (CMBI) are generated.

There is no significant difference in a boil point and other physical properties between the monoisocyanate and aromatic isocyanate including xylylene diisocyanate, and therefore, the purification step requires harsh conditions, reduced pressure and high temperature distillation.

Further, as the time of exposure to high temperature in the purification step increases, various byproducts, such as dimers, trimers, etc., are additionally generated, which reduces the yield of aromatic isocyanate.

Accordingly, the present invention enables preparation of a high-purity isocyanate composition by suppressing the generation of by-products using a stabilizer in the purification step even when a distillation step is performed at a high temperature under reduced pressure.

The amine-based compound that may be used in the present invention is not particularly limited as long as it is a compound having an amino group in the molecule. Specifically, it may be a compound including an amino group together with a ring structure in the molecule, and more specifically, it may be an amine including a bifunctional or higher ring structure, including two or more amino groups in the molecule. Specific examples of the amine-based compound may include one or more selected from the group consisting of phenylene diamine, 1,2-xylylene diamine (o-xylylene diamine), 1,3-xylylene diamine (m-xylylene diamine), 1,4-xylylene diamine (p-xylylene diamine), $\alpha,\alpha,\alpha',\alpha'$-tetramethylxylylene diamine, toluene diamine, diphenylmethane diamine, 1,2-bis(aminomethyl)cyclohexane, 1,3-bis(aminomethyl)cyclohexane, 1,4-bis(aminomethyl)cyclohexane, 1,4-tetramethylene diamine, 1,5-pentamethylene diamine, and 1,6-hexamethylene diamine, and isophorone diamine.

Among the amine-based compounds, xylylene diamine (XDA) or a salt thereof may exhibit a better effect when applied to the method of preparing isocyanate. Preferably, the amine-based compound or the salt thereof may include one or more selected from the group consisting of m-xylylene diamine, p-xylylene diamine, o-xylylene diamine, and salts thereof. More specifically, it may be m-xylylene diamine, p-xylylene diamine or o-xylylene diamine and XDA hydrochloride salt or XDA carbonate salt, and one or more compounds thereof may be used.

Meanwhile, in the method including the reaction step of one embodiment, a salt of the amine-based compound may be used to suppress a rapid reaction with phosgene. The salt of the amine-based compound may be prepared in advance by a neutralization reaction of reacting the amine-based compound with anhydrous hydrochloric acid or carbonic acid, before the reaction step. The neutralization reaction for forming the salt may be performed at a temperature corresponding to or lower than the reaction step. For example, the neutralization reaction may be performed at a temperature of 20°C to 80°C.

In addition, according to one embodiment of the present invention, the reaction step may further include an organic solvent selected from the group consisting of a halogenated hydrocarbon-based organic solvent, an ester-based organic solvent, and a mixture thereof. The organic solvent may have a boiling point of 120°C or higher, more specifically, a boiling point of 120°C to 250°C. When the reaction is performed in a solvent having such a high boiling point, a high-purity isocyanate may be prepared in a high yield.

The halogenated hydrocarbon-based organic solvent may be specifically a halogenated aromatic hydrocarbon-based organic solvent such as monochlorobenzene, 1,2-dichlorobenzene, or 1,2,4-trichlorobenzene, etc.

Further, the ester-based organic solvent may be specifically fatty acid esters such as amyl formate, n-butyl acetate, isobutyl acetate, n-amyl acetate, isoamyl acetate, methylisoamyl acetate, methoxybutyl acetate, sec-hexyl acetate, 2-ethylbutyl acetate, 2-ethylhexyl acetate, cyclohexyl acetate, methylcyclohexyl acetate, benzyl acetate, ethyl propionate, n-butyl propionate, isoamyl propionate, ethyl acetate, butyl stearate, butyl lactate or amyl lactate, etc.; and aromatic carboxylic acid esters such as methyl salicylate, dimethyl phthalate or methyl benzoate, etc.

More specifically, the organic solvent may include at least one of a halogenated aromatic hydrocarbon-based organic solvent and an ester-based organic solvent having a boiling point of 120°C or higher, or 120°C to 250°C, among the above-mentioned halogenated hydrocarbon-based organic solvents and ester-based organic solvents.

When the phosgenation reaction is performed in the organic solvent as described above, the amine-based compound or the salt thereof may be used at a concentration of 20% by volume or less. When the concentration of the amine-based compound or the salt thereof exceeds 20% by volume, there is a concern about precipitation of a large amount of amine hydrochloride salt.

Meanwhile, according to one embodiment of the present invention, the reaction step may be carried out at 100°C to 200°C, preferably at 120°C to 150°C. When the reaction step is carried out at lower than 100°C, the amount of heat required for the reaction may not be sufficiently supplied, and thus the reaction may not proceed or may proceed very slowly, and when the reaction is carried out at higher than 200°C, the process stability may reduce due to rapid reaction progress, and the by-product production rate may increase due to excessive side reactions.

Meanwhile, the composition prepared through the above reaction step includes EBI, CMBI and other by-products in addition to isocyanate as described above, and therefore, an additional purification process is required to obtain the desired product, isocyanate. Accordingly, the present invention includes a purification step of separating the isocyanate from the composition. The purification step may be, for example, distillation, but is not particularly limited as long as it is a possible purification method.

Meanwhile, distillation of isocyanate is usually carried out at high temperatures, and boiling points of isocyanate and the byproducts thereof are similar, and there is a high possibility that additional byproducts are generated during distillation at high temperatures. In addition, as the time of exposure to high temperature in the purification step increases, various byproducts, such as dimers, trimers, etc., are additionally generated, which reduces the yield of isocyanate.

Therefore, during a long-time distillation process, there may be problems that additional byproducts are generated due to the decomposition and side reactions of isocyanate, and discoloration such as coloring and cloudiness occurs. In addition, the higher the content of such byproducts, the more discoloration occurs when manufacturing optical products based on them.

According to one embodiment of the present invention, the purification step may be performed in the presence of a stabilizer.

As described, when the purification step is performed in the presence of a stabilizer, additional by-product generation may be suppressed even though distillation is carried out for a long time. The stabilizer is mixed with isocyanate in the purification step to play a role in suppressing side reactions of isocyanate at high temperatures by radical capture reaction.

Meanwhile, when a stabilizer, etc. is added in the reaction step, it plays a role in suppressing the generation of a by-product monoisocyanate by promoting the forward reaction and suppressing the side reactions. In contrast, as in the present invention, when the purification step of isocyanate after the reaction is carried out in the presence of a stabilizer, it has a unique effect of improving the yield by minimizing reactions between isocyanates and other side reactions even at high temperatures.

In addition, some stabilizers may have limitations in use due to problems in that they are not easily mixed or dissolved simply by adding the stabilizers to final products. However, when a stabilizer is included in the purification step as in the present invention, the solubility of the stabilizer increases, allowing the use of various types of stabilizers, and the corresponding stabilizer remains even after the preparation is completed, and thus the storage stability of the isocyanate composition finally prepared may also be improved.

As described, the purification step in the present invention is carried out in the presence of a stabilizer, thereby increasing process stability and contributing to improving the efficiency of the overall process by obtaining a high-purity isocyanate composition.

Meanwhile, according to one embodiment of the present invention, the stabilizer may include one or more selected from the group consisting of a phosphorus-based compound, a piperidine-based compound, and a sulfone-based compound.

The phosphorus-based compound may include phosphoric acid, phosphorous acid, phosphonous acid, phosphonic acid, and esters thereof, etc. Specific examples may include phosphoric acid, pyrophosphoric acid, metaphosphoric acid, polyphosphoric acid, bis(2,4-di-tert-butylphenyl)pentaerythritol diphosphite, dibutyl phosphate, triphenyl phosphite, trisnonylphenyl phosphite, phenylphosphinic acid, ethyl phosphate, bis(2-ethylhexyl) phosphate, 1,4-butylenediphosphonic acid, isopropyl phosphate, 3-phosphonopropionic acid, etc., and any one thereof or a mixture of two or more thereof may be used.

The piperidine-based compound refers to a cyclic compound and a derivative thereof having a heteroatom in the molecular structure. Specific examples of the piperidine-based compound may include 2,2,6,6-tetramethylpiperidine-1-oxyl (TEMPO), 4-hydroxy-2,2,6,6-tetramethylpiperidine 1-oxyl (hereinafter, referred to as 4-hydroxy TEMPO), 4-methoxy-2,2,6,6-tetramethyl-piperidine 1-oxyl (4-methoxy-TEMPO), 2,2,6,6-tetramethyl-4-benzyloxypiperidine 1-oxyl (4-benzyloxy-TEMPO), or 4-acetamido-2,2,6,6-tetramethylpiperidine 1-oxyl (hereinafter, referred to as 4-acetamido TEMPO), etc., and any one thereof or a mixture of two or more thereof may be used. Specifically, it may be TEMPO, 4-hydroxy TEMPO, or 4-acetamido TEMPO, and any one thereof or a mixture of two or more thereof may be used.

The sulfone-based compound may include 4,4'-biphenyldisulfonic acid, methyl-p-toluene sulfonate, dodecyl benzenesulfonic acid (4-dodecylbenzenesulfonic acid), p-toluene sulfonic acid, p-toluene sulfonamide, benzene sulfonamide, methane sulfonamide, and p-toluenesulfonyl isocyanate, and any one thereof or a mixture of two or more thereof may be used.

Further, halogenated acetic acid, benzoyl chloride, sulfuric acid, sulfuric acid ester, an ion exchange resin, a chelating agent, etc. may be further included, in addition to the above-mentioned phosphorus-based compounds, piperidine-based compounds, and sulfone-based compounds.

Meanwhile, the content of the stabilizer may be 50 ppm to 5000 ppm or less, preferably, 100 ppm to 3000 ppm, or 300 ppm to 2500 ppm, or 500 ppm to 2000 ppm, based on the weight of the isocyanate composition. When the concentration is 50 ppm or less, it is difficult to verify the effects of the stabilizer, such as suppression of by-product generation, and when it exceeds 5000 ppm, the effect according to the increased addition amount is not significant, and there may be a problem of increased preparation costs due to reasons of additional purification, etc.

Meanwhile, according to one embodiment of the present invention, the reaction step or the purification step may further include a side reaction inhibiting additive. Specific examples of the side reaction inhibiting additive may include 2,2,6,6-tetramethylpiperidine-1-oxyl (TEMPO), 4-hydroxy-2,2,6,6-tetramethylpiperidine 1-oxyl (4-hydroxy TEMPO), 4-acetamido-2,2,6,6-tetramethylpiperidine 1-oxyl (4-acetamido TEMPO), etc., and any one thereof or a mixture of two or more thereof may be used. When the material is introduced at the beginning of the reaction step, it promotes the forward reaction and suppresses the side reactions by eliminating hydrogen of amine or carbamoyl chloride, which is an intermediate product, during the phosgenation reaction, and thus plays a role in suppressing the generation of monoisocyanate, which is a by-product.

The purification step may be performed by a method which is generally used for purifying isocyanate compounds. In one embodiment, the purification step may be performed by reduced pressure distillation using a distillation tower and/or thin film distillation.

As the distillation tower used in the reduced pressure distillation, a plate tower or a packed tower may be used without limitation. The number of theoretical plates of the distillation tower may be, for example, 2 or more, or 5 or more, or 7 or more, and 60 or less, or 40 or less.

The temperature during the reduced pressure distillation is maintained at lower than 180°C, specifically, at 100°C or higher, or 130°C or higher, and the pressure during the reduced pressure distillation may be 0.001 kPa or more, or 0.005 kPa or more, or 0.01 kPa or more, or 0.1 kPa or more, and 50 kPa or less, or 45 kPa or less, or 40 kPa or less. The temperature and pressure during the reduced pressure distillation refer to the bottom temperature of the distillation tower.

In addition, the top temperature of the tower during the reduced pressure distillation may be 10°C or higher, or 20°C or higher, and 180°C or lower, or 160°C or lower, and the pressure may be 0.001 kPa or more, or 0.005 kPa or more, or 0.01 kPa or more, and 50 kPa or less, similar to the bottom pressure of the tower.

The thin film distillation may be performed using a thin film distillation device having an evaporator, a condenser, and a pressure reducing means. A rotation speed of a rotor of the thin film distillation device may be 50 rpm or more, or 120 rpm or more, or 200 rpm or more, and 500 rpm or less, 400 rpm or less, or 350 rpm or less. The temperature during thin film distillation is maintained at lower than 200°C, for example, 100°C or higher, or 110°C or higher, and 190°C or lower, or 180°C or lower, or 170°C or lower, and the pressure may be 0.001 kPa or more, and 50 kPa or less, 30 kPa or less, 10 kPa or less, or 1 kPa or less.

Under the above-described reduced pressure distillation conditions and/or thin film distillation conditions, the residence time of the purification step may be reduced while increasing the purification efficiency, thereby obtaining a high-purity isocyanate compound.

Meanwhile, the purification step may be performed in multiple stages while varying the temperature and/or pressure conditions. For example, the purification step may be performed by including the steps of removing solvents, removing low-boiling-point impurities, and removing oligomers.

Specifically, the purification step may be performed at a temperature of lower than 180°C, or a temperature of 120°C to 170°C and a pressure of 0.001 kPa to 50 kPa, wherein reduced pressure distillation for solvent removal is performed under a first temperature and a first pressure, then low-boiling-point impurities are removed under a second temperature and a second pressure, and then high-boiling-point impurities are removed under a third temperature and a third pressure using a thin film distillation device. Meanwhile, the order of the above purification steps may be changed.

In addition, before the reduced pressure distillation step, a nitrogen bubbling step may be further performed to remove unreacted phosgene and hydrogen chloride gas inside the reactor where the phosgenation reaction is completed.

Meanwhile, the final isocyanate composition obtained through the purification step may include the stabilizer of the present invention which is used in the purification step, as well as the side reaction inhibitor, the isocyanate oligomer, etc. Among them, the stabilizer is partially removed during the purification process, or the remaining stabilizer or additive that is not removed may be included, and the final isocyanate may be included in an amount of about 99% or less, or about 95% or less of the addition amount of the stabilizer or additive.

Accordingly, the present invention provides an isocyanate composition including the isocyanate, the isocyanate oligomer, and the stabilizer.

According to one embodiment of the present invention, the content of any one or more of P and S derived from the stabilizer may be 2000 ppm or less, based on the weight of the isocyanate composition. Meanwhile, with regard to the type and role of the stabilizer, reference may be made to the above-described contents. In other words, the isocyanate composition of the present invention includes the stabilizer in a predetermined range of content, thereby preventing conversion of the isocyanate into the isocyanate oligomer, and thus it has excellent preservation/storage stability.

Preferably, the content of any one or more of P and S derived from the stabilizer or the additive may be 1800 ppm or less, 1500 ppm or less, 1200 ppm or less, 1000 ppm or less, 800 ppm or less, 500 ppm or less, or 400 ppm or less, and 10 ppm or more, 20 ppm or more, 30 ppm or more, or 35 ppm or more, based on the weight of the isocyanate composition.

Meanwhile, the content of any one or more of P and S derived from the stabilizer may be measured by a method such as inductively coupled plasma atomic emission spectroscopy (ICP-AES), inductively coupled plasma mass spectrometry (ICP-MS), which will be specified in Examples described below. For example, P may be derived from the above-described phosphorus-based compound, and S may be derived from a sulfone-based compound, respectively.

In addition, the isocyanate composition according to one embodiment may include the stabilizer in an amount of 50 ppm to 3000 ppm, based on the weight of the isocyanate composition. As described above, the stabilizer may be partially included in the final isocyanate composition to form the isocyanate composition. In addition, the content of the stabilizer may be calculated by measuring the content value of the stabilizer-derived elements in the above-described isocyanate composition. Preferably, the stabilizer may be included in an amount of 100 ppm or more, 200 ppm or more, 300 ppm or more, or 400 ppm or more, and 3000 ppm or less, 2500 ppm or less, or 2000 ppm or less, based on the weight of the isocyanate composition.

Meanwhile, the type of the stabilizer may refer to those described in the above-described method of preparing isocyanate. For example, the stabilizer may include one or more selected from the group consisting of pyrophosphoric acid, metaphosphoric acid, polyphosphoric acid, bis(2,4-di-tert-butylphenyl)pentaerythritol diphosphite, dibutyl phosphate, methyl-p-toluene sulfonate, 4-dodecylbenzenesulfonic acid, p-toluene sulfonic acid, p-toluene sulfonamide, and benzene sulfonamide.

Meanwhile, the isocyanate composition of the present invention may include the stabilizer as described above. In this regard, the stabilizer may be added at any step during the step of preparing the isocyanate composition, for example, before and/or after the isocyanate purification step, or during the purification step. When added during the purification step, the isocyanate composition of the present invention may be prepared according to the preparation method, as described above, including a reaction step of preparing a composition including an isocyanate by reacting an amine-based compound or a salt thereof with phosgene; and a purification step of separating the isocyanate from the composition, wherein the purification step is performed in the presence of a stabilizer.

Further, the isocyanate composition according to one embodiment has an isocyanate oligomer content of 2% or less, based on the weight of the isocyanate composition, after being stored for 100 days at 5°C. Preferably, the oligomer content is 1.9% or less, 1.8% or less, or 1.7% or less, based on the weight of the isocyanate composition, after being stored for 100 days at 5°C. Meanwhile, the 'oligomer' refers to a material prepared from isocyanate and/or by-products after the purification step, for example, a material having a molecular weight of about 600 g/mol to about 2000 g/mol which is formed by combining the same. Meanwhile, the theoretical lower limit of the oligomer content in the isocyanate is 0 wt%, but isocyanate with high reactivity may generate oligomers as dimers or trimers during the preparation process or during storage due to binding between isocyanates, and for example, it may be 0.01 wt% or more, 0.05 wt% or more, 0.1 wt% or more, or 0.5 wt% or more. A specific method of analyzing the oligomers will be specified in Examples described below.

In addition, the present invention provides a polyisocyanate composition including a polyisocyanate which is formed by a polymerization reaction of the isocyanate composition of the present invention and a polyvalent thiol.

The polyvalent thiol is a compound containing two or more thiol groups (-SH) in the molecule, and specifically, may be a compound having two or more, or three or more, and eight or less, or five or fewer thiol groups in the molecule. The polyvalent thiol may be, for example, 2,3-bis(2-sulfanyl ethyl sulfanyl)propane-1-thiol.

The polymerization reaction is performed by a thiourethane reaction between the isocyanate compound in the isocyanate composition and the thiol group in the polyvalent thiol. The polymerization reaction may be performed under normal pressure and an inert gas atmosphere such as nitrogen, argon, etc. In addition, the polymerization reaction may be performed in the presence of a catalyst or in the absence of a catalyst. As a result of the polymerization reaction, polyisocyanate is produced, and the polyisocyanate composition including the same may further include additives such as an internal release agent, an ultraviolet absorber, a polymerization initiator, a thermal stabilizer, a color corrector, a chain extender, a crosslinking agent, a light stabilizer, a filler, and a photosensitizer, as needed.

Further, the present invention provides an article including the above-described polyisocyanate composition. The article may be an optical material product, specifically, an optical article such as a spectacle lens, a camera lens, a plastic lens, a prism, etc.

[Effect of the Invention]

According to a method of preparing an isocyanate composition of the present invention, an isocyanate composition having high purity and improved storage property may be prepared by suppressing thermal denaturation and by-product generation during a purification process.

[Detailed Description of the Embodiments]

Hereinafter, the actions and effects of the present invention will be described in more detail with reference to specific exemplary embodiments of the present invention. However, these exemplary embodiments are provided only for illustrating the present invention, but the scope of the present invention is not limited thereby.

**<Example>**

**Example 1**

1,2-dichlorobenzene (471 g) and m-xylylene diamine (XDA, 32.5 g) were put in a flask, and stirred while injecting anhydrous hydrochloric acid at a speed of 20 g/hr at room temperature (23±5°C).

The temperature increased to 50°C while injecting anhydrous hydrochloric acid. After injection for 4 hours, the generated salt was cooled to room temperature, and phosgene (43 g) was introduced into the reactor, and the reactor temperature was heated to 130°C.

From the time of phosgene injection to the end of the reaction, a dry ice-acetone cooler was used to prevent phosgene from leaking out.

After the reactor temperature reached 130°C, the reactor temperature was maintained at 125°C to 135°C for 2 hours so that the reaction solution became transparent. After the solution became transparent, the inside of the reactor was cooled to 80°C and cooled while introducing nitrogen.

The solvent was removed through reduced pressure distillation from the reaction solution from which phosgene was removed, and bis(2,4-di-tert-butylphenyl) pentaerythritol diphosphite (1000 ppm, based on the weight of the isocyanate composition) was added to the reactor, and a product was distilled using a thin film distillation device with a rotor rotating at 200 rpm at 0.1 kPa in a thin film distillation device at 150°C to remove high-boiling point impurities, thereby preparing an isocyanate composition including m-XDI.

**Example 2**

An isocyanate composition including m-XDI was prepared in the same manner as in Example 1, except that dibutyl phosphate (1000 ppm) was added instead of bis(2,4-di-tert-butylphenyl) pentaerythritol diphosphite.

**Example 3**

An isocyanate composition including m-XDI was prepared in the same manner as in Example 1, except that trisnonylphenyl phophite (1000 ppm) was added instead of bis(2,4-di-tert-butylphenyl) pentaerythritol diphosphite.

**Example 4**

An isocyanate composition including m-XDI was prepared in the same manner as in Example 1, except that benzene sulfonamide (1000 ppm) was added instead of bis(2,4-di-tert-butylphenyl) pentaerythritol diphosphate.

**Example 5**

An isocyanate composition including m-XDI was prepared in the same manner as in Example 1, except that p-toluene sulfonamide (2000 ppm) was added instead of bis(2,4-di-tert-butylphenyl) pentaerythritol diphosphate.

**Example 6**

An isocyanate composition including m-XDI was prepared in the same manner as in Example 1, except that p-toluene sulfonamide (500 ppm) was added instead of bis(2,4-di-tert-butylphenyl) pentaerythritol diphosphate.

**Example 7**

An isocyanate composition including m-XDI was prepared in the same manner as in Example 1, except that 1,2-dichlorobenzene (471 g), m-xylylene diamine (XDA, 32.5 g), and 4-hydroxy-2,2,6,6-tetramethyl piperidine 1-oxyl (4-hydroxy TEMPO, 0.24 g) were put in a flask, and stirred while injecting anhydrous hydrochloric acid at a speed of 20 g/hr at room temperature (23±5°C), thereby preparing a salt.

**Comparative Example 1**

An isocyanate composition including m-XDI was prepared in the same manner as in Example 1, except that bis(2,4-di-tert-butylphenyl) pentaerythritol diphosphate was not added in the purification step.

**Comparative Example 2**

An isocyanate composition including m-XDI was prepared in the same manner as in Example 7, except that bis(2,4-di-tert-butylphenyl) pentaerythritol diphosphate was not added in the purification step.

**<Experimental Example>**

(1) XDI purity

The isocyanate compositions prepared in Examples and Comparative Examples were analyzed using GC. GC used in the analysis was HP-6890, and FID was used for detection. A used column was DB-17 (30 m * 0.25 mm * 0.5 μm), a carrier gas was nitrogen (1.0 mL/min), an oven temperature was 80°C -> 5°C/min -> 160°C (8 min) -> 20°C/min -> 280°C (18 min).

(2) Loss rate (%)

For the isocyanate compositions prepared in Examples and Comparative Examples, the loss rate was determined according to Equation 1 below using the weight of m-XDI before thin film distillation and the weight of m-XDI obtained after thin film distillation.

Loss rate (%) = 100 - [(Weight of m-XDI after thin film distillation/ Weight of m-XDI before thin film distillation) × 100]     [Equation 1]

(3) Analysis of contents of elements derived from stabilizer

Pretreatment of sample for ICP-AES analysis

0.3 g of the sample and 5 ml of $HNO_3$ were put in a quartz vial, and heated at 240°C for 30 minutes using Multiwave 7000 to obtain a clear solution, followed by mass up to 40 mL using DIW in a falcon tube, and then analyzed. The dilution rate of the sample was about 130-fold.

Calibration curve preparation

P, S standard solutions (1000 ug/ml Metals in $H_2O$) from SCP Science were used as standards for preparing calibration curves of P and S elements. The standard solution prepared by diluting a reagent blank and an ICP/AES standard solution was measured using ICP-AES, and the calibration curves were prepared with concentrations of about 0.08 ppm to about 10 ppm for P element and about 0.4 ppm to about 10 ppm for S element. At this time, 214.914 nm and 181.975 nm were selected as the characteristic wavelengths of P and S elements, respectively, and it was confirmed that the correlation coefficient of the calibration curve was 0.999 or higher. After analyzing the sample, the intensity at each element's wavelength was substituted into the calibration curve to calculate the content value, which was multiplied by the dilution rate to calculate the final result value (ppm). In addition, the content of the stabilizer in the isocyanate composition was calculated, based on the above result values.

(4) Oligomer content (%)

The oligomer content of the isocyanate composition was measured by the following method.
First, the isocyanate compositions prepared in Examples and Comparative Examples were stored for 100 days at 5°C, and then gel permeation chromatography (GPC) analysis was performed on each isocyanate composition under the following conditions to obtain a molecular weight distribution curve (GPC curve) for the isocyanate composition, and the area of the fraction corresponding to the oligomer in the total area under the GPC molecular weight distribution curve was expressed as a percentage. Specifically, the oligomer content (area%) was calculated according to Equation 2 below.

$$[Equation\ 2]$$

$$Oligomer\ content\ (\%) = [B/A]\ X\ 100$$

in Equation 2,

A represents the total area under the curve in the molecular weight distribution curve (GPC curve) for the isocyanate composition, and
B represents the area of the peak corresponding to the oligomer in the molecular weight distribution curve for the isocyanate composition.

The total area of the GPC curve and the area of the fraction corresponding to the oligomers are each obtained through integration. At this time, the oligomers were evaluated, based on a polymer having a weight average molecular weight (Mw) value of 600 g/mol to 2000 g/mol, and the fraction corresponding to the oligomers in the GPC curve is $19.42 \leq logMw \leq 22.18$.

<GPC analysis conditions>

Device used: Waters 2988
Column: Shodex KF-801, KF-802, KF-803 300mm
Sample concentration: prepared by dissolving 0.1 mg of 1 wt/vol% sample in 9.9 ml of tetrahydrofuran (THF)
Carrier: THF
Detection method: UV
Flow rate: 1.0 ml/min
Column temperature: 40°C
Molecular weight when preparing the calibration curve: polystyrene of 1000 g/mol to 20,000 g/mol was used.

(5) Degassing time during lens manufacturing

Lens manufacturing

Each of the isocyanate compositions (41.6 g) prepared in Examples and Comparative Examples, zelec UN (manufactured by Stepan, 0.08 g), and biosorb 583 (manufactured by Sakai Chemical industry Co., Ltd, 0.08 g) were put in a flask, and mixed with stirring at room temperature for about 20 minutes to prepare polyisocyanate.
After visually confirming that each component was well mixed with the polyisocyanate, dibutyltin chloride (0.004 g) was added as a catalyst and mixed with stirring for 10 minutes.
2,3-bis(2-sulfanyl ethyl sulfanyl)propane-1-thiol (38.4 g) was added to the resulting mixture, and degassed under 5 mbar condition and stirred for a predetermined period of time to prepare a polymerizable composition.
The above polyisocyanate composition was filtered through a 1 μm polytetrafluoroethylene (PTFE) filter, and then injected into a mold made of a glass mold and tape. The mold was placed in an oven, and the temperature of the oven was gradually increased from 10°C to 120°C, and a polymerization reaction was performed for about 20 hours.
After polymerization was completed, the mold was taken out from the oven and released to obtain a plastic lens. The obtained lens was annealed at 120°C for 6 hours.

Measurement of degassing time

In the above process of manufacturing the lens, the composition for polymerizing polyisocyanate was degassed under 5 mbar condition, and the time (min) taken from the start of degassing to the point where bubbles were no longer generated was measured, and from the results, the effect of improving workability was evaluated.

[Table 1]

|  | XDI purity (%)* | Loss rate (%) | Content of element derived from stabilizer (ppm) | Stabilizer content (ppm) | Oligomer content (%) | Degassing time upon manufacturing lens (min) |
|---|---|---|---|---|---|---|
| Example 1 | 99.38 | 19.6 | P 91 | 888 | 1.26 | 120 |
| Example 2 | 99.54 | 19.0 | P 140 | 950 | 1.05 | 110 |
| Example 3 | 99.45 | 20.5 | P 37 | 823 | 1.11 | 120 |
| Example 4 | 99.68 | 18.8 | S 193 | 948 | 1.4 | 130 |
| Example 5 | 99.59 | 18.1 | S 357 | 1910 | 1.1 | 110 |
| Example 6 | 99.75 | 18.9 | S 82 | 438 | 1.21 | 120 |
| Example 7 | 99.82 | 17.2 | P 87 | 849 | 0.98 | 110 |
| Comparative Example 1 | 99.19 | 23.5 | ND** | - | 3.39 | 150 |
| Comparative Example 2 | 99.50 | 21.4 | ND** | - | 2.10 | 145 |
| * area% upon GC analysis<br>** Not Detected | | | | | | |

As confirmed in Table 1, when the distillation step was performed by adding the stabilizer in the distillation step, a high-purity isocyanate composition was obtained, and it was confirmed that a high-purity isocyanate composition with improved productivity may be prepared by reducing the loss of XDI before and after the thin-film distillation. It was also confirmed that the isocyanate composition including the corresponding stabilizer has improved storage stability because oligomer formation is reduced even when stored for a long time, and the degassing time is reduced during lens manufacturing, leading to the effect of improving workability.

**Claims**

1.    A method of preparing an isocyanate composition, the method comprising:

    a reaction step of preparing a composition including an isocyanate by reacting an amine-based compound or a salt thereof with phosgene; and
    a purification step of separating the isocyanate from the composition,
    wherein the purification step is performed in the presence of a stabilizer.

2.    The method of claim 1, wherein the amine-based compound or the salt thereof includes one or more selected from the group consisting of phenylene diamine, 1,2-xylylene diamine, 1,3-xylylene diamine, 1,4-xylylene diamine, $\alpha,\alpha,\alpha',\alpha'$-tetramethylxylylene diamine, toluene diamine, diphenylmethane diamine, 1,2-bis(aminomethyl)cyclohexane, 1,3-bis(aminomethyl)cyclohexane, 1,4-bis(aminomethyl)cyclohexane, 1,4-tetramethylene diamine, 1,5-pentamethylene diamine, 1,6-hexamethylene diamine, isophorone diamine, and salts thereof.

3.    The method of claim 1, further comprising the step of forming a salt of the amine-based compound by reacting the amine-based compound with hydrochloric acid or carbonic acid, before the reaction step.

4.    The method of claim 1, wherein the reaction step is performed in the presence of an organic solvent selected from the group consisting of a halogenated hydrocarbon-based organic solvent, an ester-based organic solvent, and a mixture thereof.

**5.** The method of claim 1, wherein the reaction step is performed at 100°C to 200°C.

**6.** The method of claim 1, wherein the stabilizer includes one or more selected from the group consisting of a phosphorus-based compound, a piperidine-based compound, and a sulfone-based compound.

**7.** The method of claim 1, wherein the stabilizer includes one or more selected from the group consisting of pyrophosphoric acid, metaphosphoric acid, polyphosphoric acid, bis(2,4-di-tert-butylphenyl)pentaerythritol diphosphite, dibutyl phosphate, methyl-p-toluene sulfonate, 4-dodecylbenzenesulfonic acid, p-toluene sulfonic acid, p-toluene sulfonamide, and benzene sulfonamide.

**8.** The method of claim 1, wherein the content of the stabilizer is 50 ppm to 5000 ppm or less, based on the weight of the isocyanate composition.

**9.** The method of claim 1, wherein the reaction step or the purification step further includes one or more selected from the group consisting of 2,2,6,6-tetramethylpiperidine-1-oxyl (TEMPO), 4-hydroxy-2,2,6,6-tetramethylpiperidine 1-oxyl (4-hydroxy TEMPO), and 4-acetamido-2,2,6,6-tetramethylpiperidine 1-oxyl (4-acetamido TEMPO).

**10.** The method of claim 1, wherein the purification step is performed by distillation at 100°C to 170°C.

**11.** The method of claim 1, wherein the purification step is performed by distillation at 0.001 kPa to 50 kPa.

**12.** The method of claim 1, wherein the purification step further includes the step of removing the solvent before adding the stabilizer.

**13.** An isocyanate composition comprising an isocyanate, an isocyanate oligomer, and a stabilizer.

**14.** The isocyanate composition of claim 13, wherein the content of any one or more of P and S derived from the stabilizer is 2000 ppm or less, based on the weight of the isocyanate composition.

**15.** The isocyanate composition of claim 13, wherein the stabilizer is included in an amount of 50 ppm to 3000 ppm, based on the weight of the isocyanate composition.

**16.** The isocyanate composition of claim 13, wherein the stabilizer includes one or more selected from the group consisting of pyrophosphoric acid, metaphosphoric acid, polyphosphoric acid, bis(2,4-di-tert-butylphenyl)pentaerythritol diphosphite, dibutyl phosphate, methyl-p-toluene sulfonate, 4-dodecylbenzenesulfonic acid, p-toluene sulfonic acid, p-toluene sulfonamide, and benzene sulfonamide.

**17.** The isocyanate composition of claim 13, wherein the content of the isocyanate oligomer is 2% or less, based on the weight of the isocyanate composition, after stored at 5°C for 100 days.

**18.** A polyisocyanate composition comprising a polyisocyanate which is formed by a polymerization reaction of the isocyanate composition of claim 13 and a polyvalent thiol.

**19.** An optical article comprising the polyisocyanate composition of claim 18.

# EP 4 570 785 A1

## INTERNATIONAL SEARCH REPORT

| | International application No. |
|---|---|
| | **PCT/KR2023/011815** |

### A. CLASSIFICATION OF SUBJECT MATTER

**C07C 263/10**(2006.01)i; **C07C 263/18**(2006.01)i; **G02B 1/04**(2006.01)i

According to International Patent Classification (IPC) or to both national classification and IPC

### B. FIELDS SEARCHED

Minimum documentation searched (classification system followed by classification symbols)

C07C 263/10(2006.01); B29D 11/00(2006.01); C07C 263/16(2006.01); C07C 263/18(2006.01); C08G 18/02(2006.01); C08G 18/38(2006.01); C08G 18/76(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이소시아네이트(isocyanate), 포스겐(phosgene), 안정제(stabilizator), 피페리딘 (piperidine), 아민(amine)

### C. DOCUMENTS CONSIDERED TO BE RELEVANT

| Category* | Citation of document, with indication, where appropriate, of the relevant passages | Relevant to claim No. |
|---|---|---|
| Y | KR 10-2217747 B1 (SKC CO., LTD. et al.) 19 February 2021 (2021-02-19)<br>See claims 1, 10 and 11, and paragraphs [0020], [0050] and [0129]. | 1-5,8-15,17-19 |
| A | | 6,7,16 |
| Y | WO 2018-070540 A1 (ASAHI KASEI KABUSHIKI KAISHA) 19 April 2018 (2018-04-19)<br>See paragraphs [0013], [0191], [0192] and [0248]. | 1-5,8-15,17-19 |
| Y | KR 10-1854429 B1 (HANWHA CHEMICAL CORPORATION) 03 May 2018 (2018-05-03)<br>See claims 1 and 3. | 9 |
| A | KR 10-2022-0095865 A (HANWHA SOLUTIONS CORPORATION) 07 July 2022 (2022-07-07)<br>See claims 1-10. | 1-19 |
| A | KR 10-2022-0044911 A (SKC CO., LTD. et al.) 12 April 2022 (2022-04-12)<br>See claims 1-10. | 1-19 |

☐ Further documents are listed in the continuation of Box C.      ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **23 November 2023** | **24 November 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/011815**

| Patent document cited in search report | | | Publication date (day/month/year) | Patent family member(s) | | | Publication date (day/month/year) |
|---|---|---|---|---|---|---|---|
| KR | 10-2217747 | B1 | 19 February 2021 | CN | 112920372 | A | 08 June 2021 |
| | | | | CN | 112920372 | B | 12 May 2023 |
| | | | | KR | 10-2021-0071367 | A | 16 June 2021 |
| | | | | KR | 10-2217752 | B1 | 19 February 2021 |
| | | | | KR | 10-2456419 | B1 | 19 October 2022 |
| | | | | US | 2021-0171443 | A1 | 10 June 2021 |
| WO | 2018-070540 | A1 | 19 April 2018 | BR | 112019007392 | A2 | 02 July 2019 |
| | | | | BR | 112019007392 | B1 | 11 October 2022 |
| | | | | CN | 109790269 | A | 21 May 2019 |
| | | | | CN | 109790269 | B | 14 June 2022 |
| | | | | CN | 112979917 | A | 18 June 2021 |
| | | | | CN | 112979917 | B | 25 April 2023 |
| | | | | EP | 3527593 | A1 | 21 August 2019 |
| | | | | JP | 6666459 | B2 | 13 March 2020 |
| | | | | TW | 201829375 | A | 16 August 2018 |
| | | | | US | 11078321 | B2 | 03 August 2021 |
| | | | | US | 11118001 | B2 | 14 September 2021 |
| | | | | US | 2020-0048403 | A1 | 13 February 2020 |
| | | | | WO | 2018-070540 | A1 | 19 April 2018 |
| KR | 10-1854429 | B1 | 03 May 2018 | CN | 110114339 | A | 09 August 2019 |
| | | | | CN | 110114339 | B | 22 October 2021 |
| | | | | EP | 3564212 | A1 | 06 November 2019 |
| | | | | EP | 3564212 | B1 | 24 November 2021 |
| | | | | JP | 2020-503325 | A | 30 January 2020 |
| | | | | JP | 6856756 | B2 | 14 April 2021 |
| | | | | US | 10611724 | B1 | 07 April 2020 |
| | | | | US | 10654796 | B2 | 19 May 2020 |
| | | | | US | 2020-0102268 | A1 | 02 April 2020 |
| | | | | WO | 2018-124526 | A1 | 05 July 2018 |
| KR | 10-2022-0095865 | A | 07 July 2022 | WO | 2022-146093 | A1 | 07 July 2022 |
| KR | 10-2022-0044911 | A | 12 April 2022 | CN | 112920082 | A | 08 June 2021 |
| | | | | CN | 112920082 | B | 23 June 2023 |
| | | | | CN | 112920084 | A | 08 June 2021 |
| | | | | CN | 112920084 | B | 17 January 2023 |
| | | | | CN | 112920374 | A | 08 June 2021 |
| | | | | CN | 112920374 | B | 30 May 2023 |
| | | | | EP | 3831806 | A1 | 09 June 2021 |
| | | | | JP | 2021-091672 | A | 17 June 2021 |
| | | | | JP | 2021-091893 | A | 17 June 2021 |
| | | | | JP | 7126217 | B2 | 26 August 2022 |
| | | | | JP | 7307440 | B2 | 12 July 2023 |
| | | | | KR | 10-2021-0071356 | A | 16 June 2021 |
| | | | | KR | 10-2021-0071401 | A | 16 June 2021 |
| | | | | KR | 10-2021-0071798 | A | 16 June 2021 |
| | | | | KR | 10-2021-0071802 | A | 16 June 2021 |
| | | | | KR | 10-2021-0071803 | A | 16 June 2021 |
| | | | | KR | 10-2021-0071811 | A | 16 June 2021 |
| | | | | KR | 10-2021-0071812 | A | 16 June 2021 |
| | | | | KR | 10-2410626 | B1 | 20 June 2022 |
| | | | | KR | 10-2424204 | B1 | 25 July 2022 |

Form PCT/ISA/210 (patent family annex) (July 2022)

## INTERNATIONAL SEARCH REPORT
### Information on patent family members

International application No.

**PCT/KR2023/011815**

| Patent document cited in search report | Publication date (day/month/year) | Patent family member(s) | | | Publication date (day/month/year) |
|---|---|---|---|---|---|
| | | KR | 10-2456416 | B1 | 19 October 2022 |
| | | KR | 10-2496434 | B1 | 06 February 2023 |
| | | KR | 10-2511797 | B1 | 21 March 2023 |
| | | US | 11702384 | B2 | 18 July 2023 |
| | | US | 11731936 | B2 | 22 August 2023 |
| | | US | 11760719 | B2 | 19 September 2023 |
| | | US | 2021-0171447 | A1 | 10 June 2021 |
| | | US | 2021-0171448 | A1 | 10 June 2021 |
| | | US | 2021-0171452 | A1 | 10 June 2021 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220100078
- KR 1020230104263